# EUROPEAN PATENT APPLICATION

(11) **EP 4 545 011 A2**
(43) Date of publication of application: **30.04.2025**
(21) Application number: 24213642.2
(22) Date of filing: 24.09.2020
(51) Int. Cl.: A61B 7/00

(54) **OBTAINING RESPIRATORY RELATED SOUNDS FROM AN AUDIO RECORDING**

(62) Divisional of application: 20198192.5
(71) Applicant: Ectosense NV, 3110 Rotselaar (BE)
(72) Inventor: Vits, Steven, 3110 Rotselaar (BE); Massie, Frederik, 3110 Rotselaar (BE); Van Pee, Bart, 3110 Rotselaar (BE)
(74) Representative: IP HILLS NV

(57) **Abstract**

A method is disclosed (100) for obtaining respiratory related sounds (160), RRSs, originating from a target patient, the method comprising the steps of obtaining an input audio recording (110, 111) of a sleeping environment of the target patient, obtaining a respiratory trace (150) of the target patient's respiration, identifying (120) RRSs (130) in the input audio recording, and selecting (140), based on the respiratory trace, from the RRSs, the RRSs (160) originating from the target patient.

## Description

### Field of the Invention

The present invention generally relates, amongst others, to a method for obtaining respiratory related sounds, RRSs, originating from a target patient.

### Background of the Invention

In the field of sleep analysis one of the elements to study are respiratory related sounds, RRSs. An RRS is a short audio fragment of a sound originating from a patient during their sleep analysis, for example a snoring sound, a sighing sound, a heavy breathing sound, or a moaning sound. Further analysis of such sounds may then be used to diagnose sleep disorders, such as sleep apnoea. It may further be desirable to count the duration of each RRS, the frequency of the RRSs, the total number of RRSs, and analyse various aspects of the RRSs.

The RRSs and related metrics may be obtained from an audio recording of the sleeping patient.

One way to obtain such an audio recording is by attaching a recording microphone on the face of a patient, as close to a patient's nose and mouth as possible. This has the advantage that external sounds and noises are mitigated by design. However, the presence of such a microphone may negatively influence the patient's sleep, and as a result the detected RRSs may not accurately reflect the natural sleep of the patient.

Alternatively, an audio recording device, for example a digital audio recording device, such as a mobile phone, or a dedicated audio recording device, may be placed further in the vicinity of the target patient. This way, the patient is not hindered by a microphone or any other device on or close to their face, resulting in a more natural sleep. However, in this case the drawback is that the RRSs of another person may be recorded onto the audio recording if the patient is not sleeping alone in the room.

It is therefore an aim of the present invention to solve or at least alleviate one or more of the above-mentioned problems. In particular, the disclosure aims at providing a method for identifying RRSs of the target patient in a relatively comfortable way without hindering the patient's natural sleep.

### Summary of the Invention

To this aim, according to a first aspect, a computer-implemented method for obtaining respiratory related sounds, RRSs, originating from a target patient is provided, the method comprising the steps of:
- obtaining an input audio recording of a sleeping environment of the target patient;
- obtaining a respiratory trace of the target patient's respiration;
- identifying RRSs in the input audio recording;
- selecting, based on the respiratory trace, from the RRSs, the RRSs originating from the target patient.

The input audio recording covers the sleeping environment of the target patient, i.e. apart from the target patient's RRSs, it may further comprise RRSs from other persons or animals and other environment sounds. The input audio recording thus comprises a plurality of the target patient's RRSs. Those are then all or partly selected during the selecting step. In order to distinguish the RRSs originating from the target patient from other sounds, the RRS sounds are selected based on a respiratory trace, i.e. a representation of the target patient's respiration as a function of time that covers the duration of the input audio recording. As the RRSs originating from the target patient are related to the target patient's respiration, there is a relation between these RRSs and the respiration. As a result, the RRSs originating from the target patient can be distinguished from other sounds in the input audio recording.

This results in a set of sounds that is free from other sounds that could negatively influence the analysis, allowing an accurate sleep analysis to be made. Further, as other sounds are filtered out, the audio recording does not need to be performed very close to the patient's mouth or chest. This means that the microphone does not suppress RRSs from the target patient, or does not cause unwanted RRSs itself.

The respiratory trace may further be obtained by techniques that are available in the art, for example by deriving the trace from a signal obtained by a polysomnograph, an electrocardiograph, an electromyograph, or a photoplethysmogram (PPG).

One step is the identification of RRSs. According to an embodiment, this step further comprises determining respiratory related sounds and non-respiratory related sounds, and discarding the non-respiratory related sounds.

In other words, the sounds that are not related to respiration are discarded from the audio recording first, resulting in a subset of sounds that are RRSs but which do not necessarily originate solely from the target patient. Based on the respiratory trace, the RRSs originating from the target patient are then selected from this subset.

According to an embodiment, the identifying comprises determining sets of sounds; wherein the sounds of a set originate from a same source; and wherein the selecting further comprises, based on the respiratory trace, selecting RRSs from a set of sounds originating from the target patient.

In other words, sounds are first divided into sets or clusters according to their origin. At that point it is not yet known which of the sets originate from the target patient. By reference to the respiratory trace, RRSs of a certain set can then be attributed to the target patient. Optionally, the identifying and discarding of non-RRSs may be performed before or after the determining of the sets.

The clustering of sounds into the sets according to their respective sources may for example be done by a trained classifier.

According to an embodiment, the selecting comprises determining a first subset of the RRSs having a high probability of originating from the target patient.

In other words, only those RRSs with a probability of originating from the target patient above a certain threshold are selected, e.g. a probability higher than 90%. This assures a low output error. Further, selecting RRSs with a high probability will typically be easy to determine, i.e., require low computing power and/or memory capacity.

Further, according to an embodiment, the selecting further comprises determining a second subset of the RRSs having a low probability of originating from the target patient, e.g. a probability lower than 10%. This second subset may then be discarded from the result.

According to an embodiment, the selecting further comprises training a classifier based on the first and/or second subset to select RRSs originating from the target patient; and selecting the RRSs originating from the target patient by the trained classifier.

In other words, the results obtained according to the first and/or second subset may be further refined by adding other RRSs that were not assigned to the first and/or second subsets. To accomplish this, a classifier is first trained with one or both the subsets to classify the RRSs as either belonging to the target patient or not. In other words, the first and/or second subset is used as labelled data. Then, the trained classifier is used to further classify the other RRSs resulting in a larger selection of RRSs originating from the target patient.

Optionally, the training of the classifier may only be performed when a number of undetermined RRSs is too high, i.e. there are still many identified RRSs that neither have a high probability or a low probability of originating from the target patient. In such case it may be useful to perform a more computationally intensive classification operation.

According to an embodiment, the determination of the first subset comprises determining audio timestamps associated with the RRSs from the input audio recording and respiratory timestamps associated with the RRSs from the respiratory trace; and determining the first subset based on the audio and respiratory timestamps.

In other words, the audio timestamps indicate the occurrence of the respective RRSs in the input audio recording and the respiratory timestamps indicate the occurrence of the respective respiratory cycles of the target patient. As the RRSs of the target patient are related to the patient's respiration, the selection can be performed based on these determined timestamps. To this end, a timestamp may by characterized by any detectable time feature such as for example an onset, a local maximum or a local minimum. This way, the selection operation is reduced to first identifying the time features and then performing operations on these time features.

One operation may be to determine time differences between the audio timestamps and respective respiratory timestamps. As the RRS of the target patient is related to their respiration, the time differences that are associated with the patient will be rather constant, while the time differences associated with other sources will be more randomly spread.

By then determining a histogram of the time differences, the ones having a high probability of belonging to the target patient will be relatively more present in the peak of the histogram and the ones having a low probability will be relatively more present in the tails of histogram.

According to a second aspect, a controller is disclosed comprising at least one processor and at least one memory including computer program code, the at least one memory and computer program code configured to, with the at least one processor, cause the controller to perform a method according to the first aspect.

According to a third aspect a computer program product is disclosed comprising computer-executable instructions for performing a method according to the first aspect when the program is run on a computer.

According to a fourth aspect a computer readable storage medium is disclosed comprising a computer program product according to the third aspect.

### Brief Description of the Drawings

Fig. 1 illustrates steps performed according to an example embodiment for selecting respiratory related sounds originating from a patient from an audio recording;
Fig. 2 illustrates steps performed according to an example embodiment for selecting respiratory related sounds originating from a patient from a plurality of respiratory related sounds and a respiratory trace;
Fig. 3 illustrates steps performed according to an example embodiment for an extended set of selected respiratory related sounds originating from a patient;
Fig. 4 illustrates steps performed according to an example embodiment for selecting respiratory related sounds originating from a patient from an audio recording;
Fig. 5 shows an illustrative plot of an audio recording with determined respiratory related sounds and a plot of a respiratory trace with respiratory related timestamps and respiratory related sound timestamps;
Fig. 6 shows another illustrative plot of an audio recording with determined respiratory related sounds and a plot of a respiratory trace with respiratory related timestamps and respiratory related sound timestamps;
Fig. 7A shows a histogram of time difference occurrences when all RRSs originate from a target patient;
Fig. 7B shows a histogram of time difference occurrences when no RRSs originate from a target patient;
Fig. 7C shows a histogram of time difference occurrences when RRSs originate from different sources; and
Fig. 8 shows a computing system suitable for performing various steps according to example embodiments.

### Detailed Description of Embodiment(s)

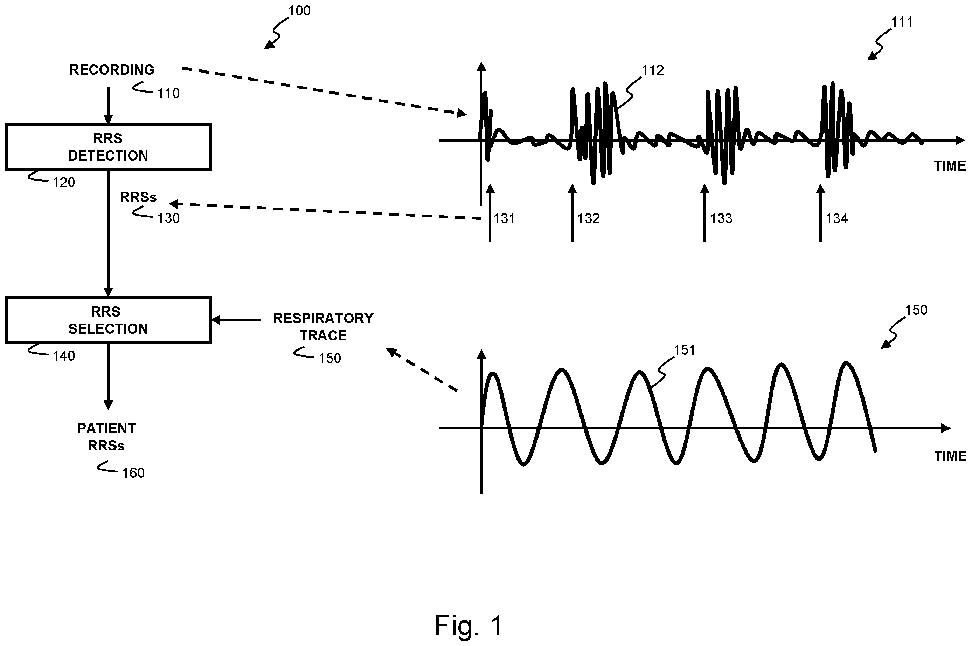

Fig. 1 shows different steps of a computer-implemented method 100 for identifying respiratory related sounds 160, RRSs, originating from a target, i.e. monitored, patient from an input audio recording 110. An RRS corresponds to an audible event generated through breathing during sleep. Such RRS may for example correspond to a snoring sound, a sighing sound, a heavy breathing sound, a moaning sound or a sound made during an apnoeic event. An RRS occurs within a breathing cycle, e.g. during inhaling, during exhaling or both. A snoring patient therefore produces a sequence of RRSs during a certain time interval, e.g. for a few seconds, minutes or even hours. Having a trace of RRSs originating from a monitored patient is valuable for performing sleep analysis as it can reveal or explain different types of health conditions.

The method starts with obtaining an audio track 110 or audio recording 110 from which the RRSs 160 originating from the patient are to be identified or selected. The audio track is recorded within audible distance from the target patient, i.e. within the patient's sleeping environment. This may for example be done by placing an audio recording device next to the patient's bed or somewhere else in the patient's bedroom. An illustrative example of such audio recording is further shown in plot 111 where the amplitude 112 of the recorded audio signal is presented as a function of time.

From this audio recording 110, the different RRSs 131-134 are identified in step 120 of method 100. These identified RRSs may relate to one specific type of RRS, e.g. only snoring, or to several or even all possible RRSs. By the identification of the RRSs, other sounds or noises are excluded from the further steps, e.g. sounds from outside the room. An RRS may for example be identified by indicating its starting time, its ending time, and/or its time period allowing to uniquely identify it within the audio recording 110.

The identification of RRSs may for example be performed by executing one or more of the following steps:
a) Determine the sound envelope of the signal 112, for example by calculating the analytical signal of the signal 112, by calculating the moving average, e.g. root mean square, RMS, value, of the signal 112, or by calculating peaks of the signal 112.
b) Determine a threshold characterizing an active sound segment. This may for example be done by calculating local signal energy values and establishing lower percentile values of local signal energy to define a baseline threshold.
c) Calculate when the sound envelope exceeds this threshold.
d) Label all episodes where the envelope exceeds the threshold as active segments.
e) Combine or remove active segments according to a set of decision rules to, for example, avoid unlikely large or small active segments.
f) Characterize the so-obtained active segments by calculating a set of features such as Mel-frequency cepstral coefficients, MFCCs, the signal power within a specific frequency range, the temporal features such as the signal mean and standard deviation, features characterizing the entropy of the signal, features characterizing the formant and pitch.
g) Identify the RRSs from the active segments, for example by a pre-trained classifier to classify all active segments as RRS or non-RRS, thereby obtaining a set of RRS segments that can originate from one or multiple sources

The identified RRSs 130 do not necessarily all originate from the target patient. For example, some of them may originate from another person sleeping next to the patient or within the same room. Also, some RRSs may originate from animals, such as from a dog sleeping in the same room. Therefore, in a subsequent selection step 140, a subset 160 of the RRSs 130 is selected as originating from the monitored patient. To do so, a respiratory trace 150 from the patient is used to select the subset 160. Such a respiratory trace characterizes the breathing of the patient during the period of the audio recording 110. Plot 151 illustrates such a trace of the patient as function of time. The rising edges may then correspond to an inhalation and the falling edges to an exhalation, or the other way around.. A respiratory trace may also correspond to discrete timestamps characterizing different breathing cycles. There is an observable temporal relationship between the trace 150 and the RRSs originating from the patient, while the other RRSs will not show such temporal relationship. Based on this the RRSs 160 originating from the patient are selected as output of step 140.

A respiratory trace may be obtained directly or derived indirectly from a measurement on the patient. For example, the trace may be derived from a signal obtained by a polysomnograph, an electrocardiograph, an electromyograph, a photoplethysmogram (PPG), or an accelerometer.

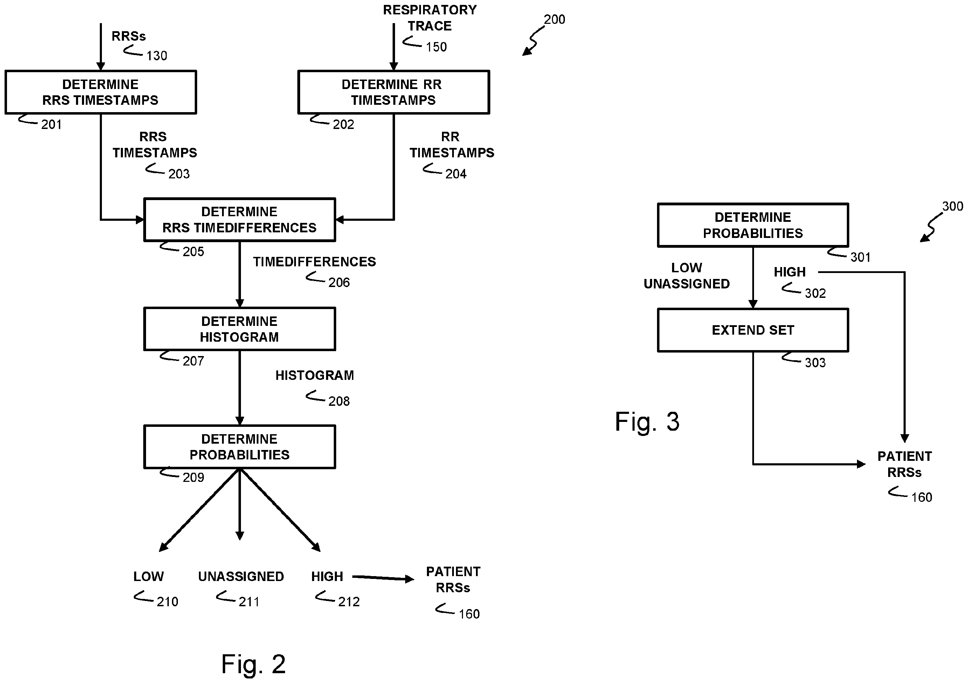

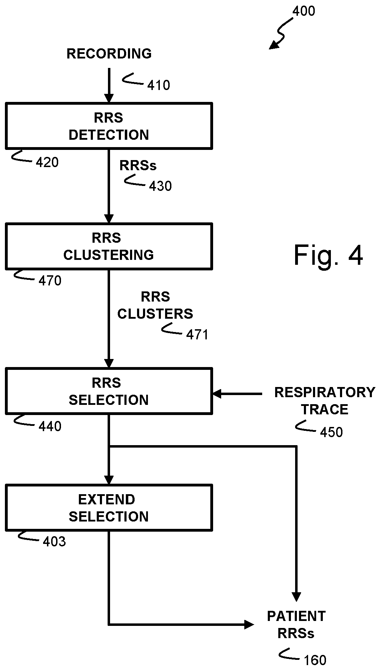

According to an embodiment, the selection 140 of RRSs 160 may be performed by the steps 200 as illustrated in Fig. 2. First, in steps 201 and 202 timestamps 203 and 204 are identified for respectively the RRSs 130 and the respiratory trace 150. For the RRSs 130, an RRS timestamp 203 may characterize the beginning of an RRS, an end of an RRS or any predetermined time reference within the occurrence of an RRS. For the respiratory trace 150, a respiratory timestamp 204 identifies a respiration cycle, for example a beginning, end or any predetermined time reference during a respiration cycle, either inhaling or exhaling. Then, in step 205, the differences 206 between the timestamps 203, 204 are determined, i.e. for each RRS timestamp 203 the time difference is determined with a nearby respiratory timestamp 204, e.g. with the next or previous respiratory timestamp. As a result, a sequence of time differences 206 is obtained wherein each time difference is associated with a respective RRS. From these time differences 206, a histogram 208 is constructed in a next step 207. Histogram 208 represents the occurrences of a certain time difference or time difference interval. In such a histogram 208, the time differences with a high occurrence show a strong temporal correlation between the associated RRSs and respiratory trace and, therefore, have a high probability of originating from the patient. Similarly, the time differences with a low occurrence show little temporal correlation between the associated RRSs and respiratory trace and, therefore, have a low probability of originating from the patient. Accordingly, the RRSs 212 having an occurrence higher than a certain first threshold are then selected as having a high probability of originating from the patient and added to the selection 160 of patient RRSs. Further RRSs 210 having an occurrence lower than a certain second threshold may then be selected as having a low probability of originating from the patient. The remaining RRSs 211 are then left as unassigned. The unassigned RRSs 211 may still be used to further extend to the set of patient RRSs 160 as further described in the embodiment with reference to Fig. 3 and Fig. 4.

Another way of selecting the patient RRSs 160 is by calculating the coherence of one or more RRSs 130 with the respiratory trace 150, i.e. the degree of synchronization between the audio signal of the one or more RRSs and the respiratory signal during the same time interval. In this case, one or more RRSs with a high coherence are considered as having a high probability of originating from the patient and one or more RRSs with a low coherence are considered as having a low probability of originating from the patient, thereby again obtaining similar sets 210, 211, 212 of RRSs. Similar to the method of Fig. 2, the RRSs 212 with a high probability are then selected as originating from the patient.

The selection of RRSs from the patient by probabilities, e.g. by the steps of Fig. 2, may be further extended depending on the outcome. For example, a considerable amount of RRSs 211 may still be unassigned, i.e. having neither a low or high probability of originating from the patient. In such a case, steps 300 as illustrated in Fig. 3 may be performed. In the first step 301, an initial selection 302 is made by selecting the RRSs with a high and/or low probability, e.g. by performing the steps 200 as described with reference to Fig. 2. Then, in step 303, further RRSs are identified as originating from the patient based on the sets of RRSs with high and/or low probabilities, e.g. sets 210 and 212. Based on these sets, some of the unassigned RRSs are further assigned as either originating from the patient or not. This step 303 can be performed in different ways. According to a first example, step 303 comprises the training of a classifier to classify RRSs according to whether they originate from the patient. For the training the RRSs with a high probability and/or with a low probability are used as labelled training data. The trained classifier is then used to add yet unassigned RRSs, e.g. RRSs 211, to the selection 160. According to a second example, an unsupervised clustering method is used to select unassigned RRSs that have a similar feature content of similar temporal coherence with RRSs from the high or low probability set. The unassigned RRSs that are clustered with the high probability set are then added to the selection 160.

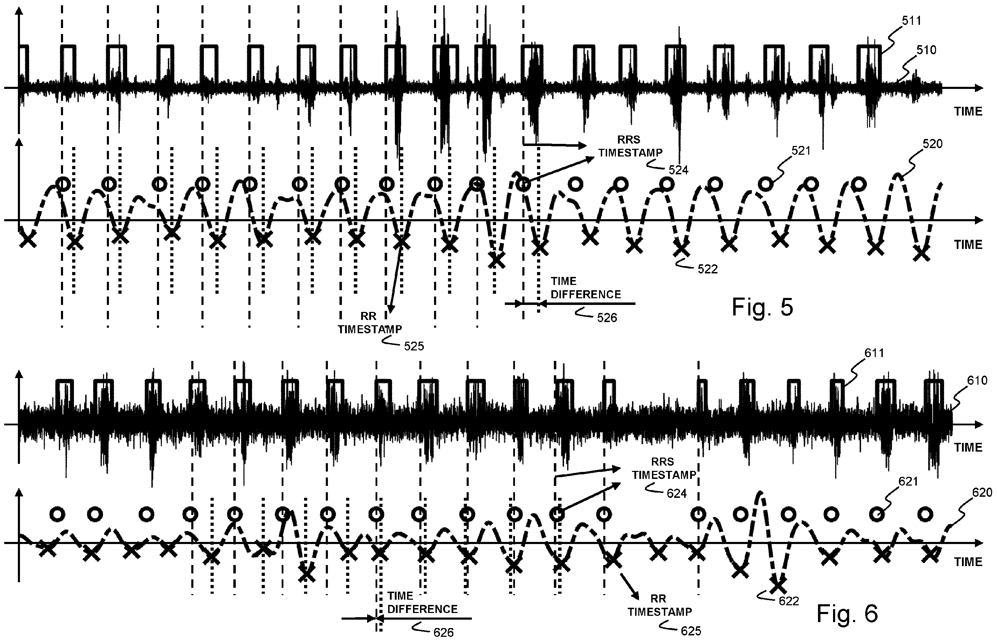

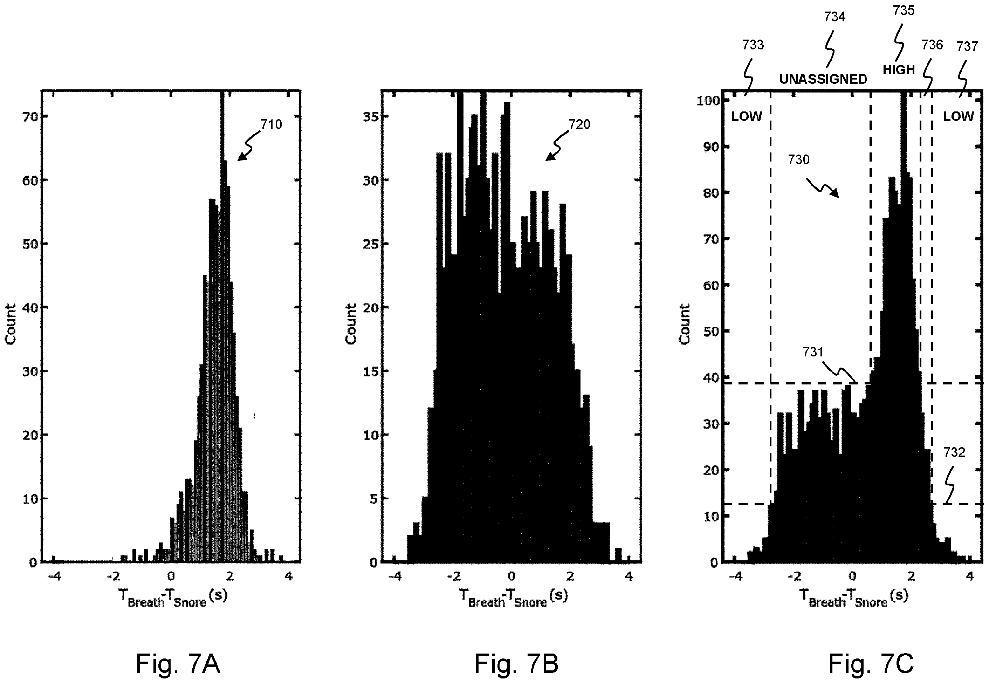

Fig. 5, 6 and 7 further illustrate the steps 200. Fig. 5 shows a first plot with the audio recording 510 together with the identified RRSs 511 as they were, for example, obtained by step 120 of Fig. 1. Fig. 5 further shows a second plot with the respiratory trace 520. In the respiratory trace 520, the onsets of the RRSs 511 are indicated with circles 521 and represent the RRS timestamps 524. In the respiratory trace 520, the periodic minima of the trace are indicated by crosses 522 and represent the respiratory related timestamps 525. The time difference 526 is then represented by the space between the dashed line representing the RRS timestamp and the previous or next dotted line representing the RR timestamp. The RRSs 511 as shown in Fig. 5 are all originating from the patient. Therefore, there is a strong temporal relationship between the RR and RRS timestamps 524, 525 which can be observed by the almost constant time differences 526. Fig. 7A then shows a histogram 710 of time differences derived from RRSs that only originate from the patient as illustrated in Fig. 5.

Similar to Fig. 5, Fig. 6 shows a first plot with the audio recording 610 together with the identified RRSs 611 as for example obtained by step 120 of Fig. 1. Fig. 6 further shows a second plot with the respiratory trace 620. In the respiratory trace 620, the onsets of the RRSs 611 are indicated with circles 621 and represent the RRS timestamps 624. In the respiratory trace 620, the periodic minima of the trace are indicated by crosses 622 and represent the respiratory related timestamps 625. The time difference 626 is then represented by the space between the dashed line representing the RRS timestamp and the closest dotted line representing the RR timestamp. The RRSs 611 as shown in Fig. 6 are not originating from the patient. Therefore, there is a weak temporal relationship between the RR and RRS timestamps 624, 625 which can be observed by the highly varying time differences 626. Fig. 7B then shows a histogram 720 of time differences derived from RRSs that only originate from the patient as illustrated in Fig. 6.

Fig. 7C then shows a histogram 730 based on time differences from both Fig. 5 and Fig. 6, i.e. a combination of histograms 710 and 720. As such, the data of histogram 730 may correspond to the histogram data 208 of method 200. As explained with reference to step 209 of Fig. 2, a first threshold 731 may then be defined in order to select RRSs with a high probability 735 and a second threshold 732 may then be defined in order to select RRSs with a low probability 733, 737. The remaining RRSs are then left unassigned as illustrated by regions 734 and 736.

According to an embodiment, a further clustering step may be performed in the method 100 as illustrated in Fig. 1. This is further illustrated with reference to the method of Fig. 4. In a first step 420 which may correspond to step 120, RRSs 430 are identified from an input audio recording 410. Then, an additional clustering step 470 is performed. In this step 470, the RRSs are grouped in a cluster when they have a high probability of belonging to the same source.

A way of clustering 470 is to first determine a set of features characterizing the RRSs, for example Mel-frequency cepstral coefficients, MFCCs, the signal power within a specific frequency range, the temporal features such as the signal mean and standard deviation, features characterizing the entropy of the RRS, features characterizing the formant and pitch. Additionally, or complementary, RRSs occurring in a temporally repetitive pattern may be identified thereby obtaining different chains of RRSs. Then the RRSs are clustered into different plausible sources based on the association with the temporal chain and/or based on the similarities between the different derived features. Clustering based on features may for example be performed by clustering algorithms such as K-means clustering and Gaussian Mixture Model, GMM, clustering. Clustering based on the obtained temporal chains may for example be performed by identifying repetitive RRS patterns that have a specific time interval between occurrences. By the clustering, RRSs may still be left unassigned, i.e. not belong to a certain source by a high probability. In such case, a further supervised clustering step can be performed. A classifier is then trained to classify RRSs into clusters by using the already clustered RRSs as labelled training data. For the classifier, a support vector machine, SVM, or neural network may be used.

The so-obtained clusters of RRSs 471 are then used as input for the further selection step 440 in which clusters with a high and/or low probability of originating from the patient are identified. The cluster with high probability are then selected as output 160. Step 440 may be performed in the same way as step 140 or as step 200 but based on clusters of RRSs instead of individual RRSs. Further, an additional step 403 may be performed wherein yet unassigned clusters of RRSs are added to the output 160 in the same way as step 303 but based on clusters of RRSs instead of individual RRSs.

The steps according to the above described embodiments may be performed by any suitable computing circuitry, for example a mobile phone, a tablet, a desktop computer, a laptop and a local or remote server. The steps according to the above described embodiments may be performed on the same device as the audio recording device. To this end, the audio recording may also be performed by for example a mobile phone, a tablet, a desktop computer or a laptop. The steps according to the above described embodiments may also be performed by a suitable circuitry remote from the environment of the patient. In such case, the audio recording may be provided to the circuitry over a communication network such as the Internet or a private network.

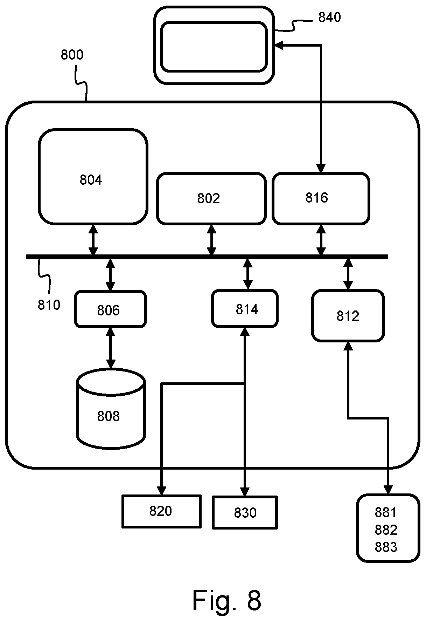

Fig. 8 shows a suitable computing system 800 comprising circuitry enabling the performance of steps according to the described embodiments. Computing system 800 may in general be formed as a suitable general-purpose computer and comprise a bus 810, a processor 802, a local memory 804, one or more optional input interfaces 814, one or more optional output interfaces 816, a communication interface 812, a storage element interface 806, and one or more storage elements 808. Bus 810 may comprise one or more conductors that permit communication among the components of the computing system 800. Processor 802 may include any type of conventional processor or microprocessor that interprets and executes programming instructions. Local memory 804 may include a random-access memory (RAM) or another type of dynamic storage device that stores information and instructions for execution by processor 802 and/or a read only memory (ROM) or another type of static storage device that stores static information and instructions for use by processor 802. Input interface 814 may comprise one or more conventional mechanisms that permit an operator or user to input information to the computing device 800, such as a keyboard 820, a mouse 830, a pen, voice recognition and/or biometric mechanisms, a camera, etc. Output interface 816 may comprise one or more conventional mechanisms that output information to the operator or user, such as a display 840, etc. Communication interface 812 may comprise any transceiver-like mechanism such as for example one or more Ethernet interfaces that enables computing system 800 to communicate with other devices and/or systems, for example with other computing devices 881, 882, 883. The communication interface 812 of computing system 800 may be connected to such another computing system by means of a local area network (LAN) or a wide area network (WAN) such as for example the internet. Storage element interface 806 may comprise a storage interface such as for example a Serial Advanced Technology Attachment (SATA) interface or a Small Computer System Interface (SCSI) for connecting bus 810 to one or more storage elements 808, such as one or more local disks, for example SATA disk drives, and control the reading and writing of data to and/or from these storage elements 808. Although the storage element(s) 808 above is/are described as a local disk, in general any other suitable computer-readable media such as a removable magnetic disk, optical storage media such as a CD or DVD, -ROM disk, solid state drives, flash memory cards, ... could be used.

As used in this application, the term "circuitry" may refer to one or more or all of the following:
(a) hardware-only circuit implementations such as implementations in only analog and/or digital circuitry and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method (100, 400) for obtaining respiratory related sounds (160, 511), RRSs, originating from a target patient, the method comprising the steps of:
obtaining an input audio recording (110, 111, 410, 510, 610) of a sleeping environment of the target patient;
obtaining a respiratory trace (150, 450, 520, 620) of the target patient's respiration;
identifying (120, 420, 470) RRSs (130, 430, 511, 611) in the input audio recording; and
selecting (140, 200, 300, 440, 403), based on the respiratory trace, from the RRSs, the RRSs (160) originating from the target patient, wherein the selecting comprises:
determining (209) a first and/or second subset of the RRSs (212, 735) having a respective high and/or low probability of originating from the target patient, and
then identifying further RRSs as originating from the target patient based on the first and/or second subset of RRSs with high and/or low probabilities.

2. The method according to claim 1, wherein the identifying comprises determining (120, 420) respiratory related sounds and non-respiratory related sounds, and discarding the non-respiratory related sounds.

3. The method according to claim 1 or 2, wherein the identifying comprises determining (470) sets of sounds (471); wherein sounds of a set originate from a same source; and wherein the selecting further comprises, based on the respiratory trace, selecting (440, 403) RRSs from a set of sounds (160) originating from the target patient.

4. The method according to any one of claims 1 to 3, wherein determining (209) a first subset of the RRSs (212, 735) having a high probability of originating from the target patient comprises constructing a histogram of time differences that are each associated with an RRS of the first subset of the RRSs, wherein each time difference of the time differences comprises a difference in time between an RRS timestamp and a timestamp of a nearby respiration cycle.

5. The method according to any one of claims 1 to 3, wherein determining (209) a first subset of the RRSs (212, 735) having a high probability of originating from the target patient comprises calculating coherence of one or more RRSs with the respiratory trace to determine degree of synchronization between the audio signal of the one or more RRSs and the respiratory signal during a same time interval.

6. The method according to any one of claims 1 to 5, wherein the selecting comprises training (303, 403) a classifier based on the first and/or second subset to select RRSs originating from the target patient; and selecting the RRSs originating from the target patient (160) by the trained classifier.

7. The method according to claim 6, wherein the selecting comprises performing the training depending on an amount of RRSs (211, 734, 736) that are not assigned to the first and second subset.

8. The method according to any one of claims 1 to 5, wherein the selecting comprises applying an unsupervised clustering process to select unassigned RRSs that have a similar feature content of similar temporal coherence with RRSs from the first or second subset.

9. The method according to claim 4, wherein the determining the first subset comprises determining (201, 202) audio timestamps (203, 521, 621) associated with the RRSs from the input audio recording (130) and respiratory timestamps (204, 522, 622) associated with the RRSs from the respiratory trace (150); and determining (205, 207, 209) the first subset based on the audio and respiratory timestamps.

10. The method according to claim 9, wherein the determining the first subset further comprises determining (206) time differences (206, 526, 625) between the audio timestamps and respective respiratory timestamps.

11. The method according to claim 10, wherein the determining the first subset further comprises determining (207) a histogram (730) of the time differences; and identifying (209) from the histogram the first subset (212).

12. The method according to any one of claims 1 to 11, wherein the respiratory trace is derived from a signal obtained by a polysomnograph, an electrocardiograph, an electromyograph, or a photoplethysmogram (PPG).

13. A controller (800) comprising at least one processor and at least one memory including computer program code, the at least one memory and computer program code configured to, with the at least one processor, cause the controller to perform the method according to any one of claims 1 to 12.

14. A computer program product comprising computer-executable instructions for performing the method according to any one of claims 1 to 12, when the program is run on a computer.

15. A computer readable storage medium comprising computer-executable instructions for performing the method according to any one of claims 1 to 12, when the computer-executable instructions are run as a program on a computer.
